## Europäisches Patentamt

## European Patent Office

## Office européen des brevets

(11) Publication number: **0 143 501**
A2

(12) # EUROPEAN PATENT APPLICATION

(21) Application number: **84201706.3**

(22) Date of filing: **22.11.84**

(51) Int. Cl.⁴: **C 07 C 118/02**

(30) Priority: **23.11.83 US 554500**

(43) Date of publication of application: **05.06.85 Bulletin 85/23**

(84) Designated Contracting States: **AT BE DE FR GB IT NL**

(71) Applicant: **STAUFFER CHEMICAL COMPANY, Westport Connecticut 06880 (US)**

(72) Inventor: **Jaffe, Fred, 130 Edgewood Road, Ossining New York 10562 (NL)**

(74) Representative: **Urbanus, Henricus Maria, Ir. et al, c/o Vereenigde Octrooibureaux Nieuwe Parklaan 107, NL-2587 BP 's-Gravenhage (NL)**

(54) **Process for preparing organic isocyanates.**

(57) This invention relates to novel processes for preparing organic isocyanates from primary monoamines and phosgene in which the primary phosgenation step is carried out at elevated temperatures from about 75°C up to about 100°C. Sophisticated mixing apparati are not required.

The primary monoamines can be aniline, a haloaniline, e.g., 3,4-dichloraniline, an alkylaniline, or a haloalkylaniline, e.g., 3-chloro-4-methylaniline.

PROCESS FOR PREPARING
ORGANIC ISOCYANATES

BACKGROUND OF THE INVENTION

1.   Field of the Invention

This invention relates to processes for preparing organic isocyanates.  More particularly, the invention relates to a process for making an organic isocyanate from a primary monoamine and phosgene in which the primary phosgenation step is carried out at elevated temperatures.

2.   Related Art

The process of preparing organic isocyanates by reacting phosgene and an amine is known.  Ordinarily, this process is carried out in two steps.  The first step of such a process is a primary or cold phosgenation step in which the amine, while suspended or dissolved in an organic solvent, is mixed with phosgene which may be added either as a gas or as a solution thereof in an inert organic solvent.  It has heretofore been found that the temperature throughout the primary phosgenation step be maintained at a point not above 60°C. in order to limit the formation of undesirable by-products such as disubstituted ureas and polyureas.  Other known processes have carried out the primary phosgenation

C-7001

step in specialized equipment that promote rapid mixing of the amine and the phosgene in order to limit formation of these undesirable by-products.

The reaction mixture from the primary phosgenation step is heated to a temperature of from about 100°C. to about 200°C. in a second or hot phosgenation step to prepare the desired organic isocyanate.

U.S. Patent No. 3,321,283 (Ewald, May 23, 1967) discloses a two step process for preparing organic isocyanates in which the first step, or primary phosgenation is carried out at temperatures from 0°C. to 200°C. and the second step, or hot phosgenation is carried out at temperatures from 100°C. to 300°C. However, the disclosed process is carried out in a very sophisticated mixing apparatus. This patent further discloses that with less efficient mixing, competing reactions will effectively lower the yield of organic isocyanates.

U.S. Patent No. 3,188,337 (Gemassmer, June 8, 1965) discloses a two step process for preparing organic isocyanates in which the first step, or primary phosgenation is carried out at temperatures not substantially above 80°C. and the second step, or hot phosgenation is carried out at temperatures from 100°C. to 300°C. However, the disclosed process is carried out by continuously pumping a liquid containing phosgene and amine through a high speed mixer at a flow rate sufficient to maintain the residence time of the reactants in the primary phosgenation step below about one minute.

U.S. Patent No. 2,795,597 (Smutz, June 11, 1957) discloses a two step process for preparing organic isocyanates in which the first step, or primary phosgenation is carried out at a temperature from 20°C. to

C-7001

70°C. and the second step, or hot phosgenation is carried out at a temperature below 150°C. to facilitate removal of trace quantities of hydrogen chloride. Although the process of this patent does not utilize a sophisticated mixing apparatus, the temperature of the primary phosgenation is kept below about 70°C., the decomposition temperature of the aromatic carbamyl chloride intermediate formed during this process step.

A process for preparing organic isocyanates in which the primary phosgenation can be carried out at elevated temperatures without formation of undesirable by-products would be advantageous. A process utilizing elevated temperatures in the primary phosgenation step and not requiring a sophisticated mixing apparatus while producing organic isocyanates in high yields without forming undesirable by-products, would be even more advantageous.

Other objects and advantages of the present invention are shown throughout this specification.

## SUMMARY OF THE INVENTION

In accordance with the present invention, a process for producing an organic isocyanate has now been discovered. This process comprises: a) charging a mixture of a primary monoamine and an inert liquid diluent into a reaction mixture comprising an inert organic liquid diluent and at least a stoichiometric excess of phosgene while maintaining the reaction temperature at from about 75°C. up to about 100°C. and maintaining a phosgene saturated liquid phase; and b) heating and refluxing the phosgene saturated reaction mixture after the charging of the primary monoamine is completed up

0143501

to about 150°C. while feeding phosgene so that the organic isocyanate is formed.

In this process the primary monoamine can be aniline, a haloaniline, e.g., 3,4-dichloroaniline, an alkylaniline, or a haloalkylaniline, e.g., 3-chloro-4-methylaniline.

## DETAILED DESCRIPTION OF THE INVENTION

The present invention comprises a novel process for producing organic isocyanates. This process comprises: a) charging a mixture of a primary monoamine and an inert liquid diluent into a reaction mixture comprising an inert organic liquid diluent and at least a stoichiometric excess of phosgene while maintaining the reaction temperature at from about 75°C. up to about 100°C. and maintaining the liquid phase saturated with phosgene; and b) heating and refluxing the phosgene saturated reaction mixture after the charging of the primary monoamine is completed up to about 150°C. while still maintaining phosgene saturation so that the organic isocyanate is formed.

The particular organic isocyanage formed is dependent upon the choice of primary monoamine. The primary monoamine employed in the processes of this invention can be varied widely. The primary monoamine can be aromatic, for example, phenyl, and can be substituted or unsubstituted. The nature of these phenyl substituents can vary widely, however, substituents that are substantially inert in this process are preferable.

Aniline and ring substituted anilines are preferred primary monoamines for this process. Haloanilines, alkylanilines and haloalkylanilines are all preferred primary monoamines. The halo-substituent can be chloride, bromide, fluoride and iodide, and can be mono

C-7001

and poly substituents of the phenyl ring. The alkyl substituent can be $C_1$ to $C_3$, can be normal and branched, and can be mono and poly substituents of the phenyl ring. The haloalkyl substituents are combinations of the halo and the alkyl substituents described above.

Illustrative of the primary monoamines used in the processes of this invention are aniline, para-chloroaniline, 3,4-dichloroaniline, m-chloroaniline, p-methylaniline, 3-chloro-4-methylaniline, 3,5-dichloro-4-methylaniline, p-bromoaniline, 3-chloro-4-isopropyl-aniline, 3-chloro-4-ethylaniline. 3,4-dichloroaniline and 3-chloro-4-methylaniline are preferred primary monoamines.

The inert liquid diluent employed in the processes of this invention can vary widely and is preferably a solvent for both the primary monoamine and the phosgene reactants. Suitable diluents are, for example, the aromatic hydrocarbons such as benzene, toluene, xylene, tetrahydronaphthalene, chlorinated aromatic hydrocarbons such as mono-chlorobenzene, ortho-dichlorobenzene, and other substituted aromatic hydrocarbons such as anisole and nitrobenzene. 1,4-dioxane is also a suitable diluent.

The processes of this invention can be carried out by first charging into a reactor the inert liquid diluent and at least a stoichiometric excess of phosgene. While saturating the liquid phase with phosgene in the reactor, the primary monoamine dissolved in the inert liquid diluent is added with agitation. Addition of phosgene is maintained during the addition of the monoamine to saturate the liquid phase and ensure an excess of phosgene, thereby avoiding formation of insoluble diaryl urea impurities.

C-7001

The temperature of the reactor is increased gradually during the addition of the primary monoamine to maintain the reaction temperature at from about 75°C. up to about 100°C. for this primary phosgenation step. Maintaining the reaction temperature at from about 75°C. up to about 85°C. is a preferred range.

When the addition of the primary monoamine is completed, the temperature of the reactor is increased up to about 150°C. while maintaining liquid phase phosgene saturation for the hot phosgenation step. This step is marked by the increased evolution of hydrogen chloride. When the evolution of hydrogen chloride ceases, the reaction is complete with the formation of the organic isocyanate.

Throughout the reaction phases of this process, phosgene saturation of the liquid phase is maintained. Phosgene is employed in excess of the stoichiometric amount required to react with the primary monoamine to form the isocyanate. Generally, at least 1.05 moles of phosgene per mole of monoamine is used, however, much larger excesses up to say 5 moles of phosgene per mole of monoamine can be used, but such large excesses are uneconomical.

The following Examples illustrate certain embodiments of the present invention but are not to be construed as limiting the invention. The scope of protection sought is set forth in the claims which follow the Examples.

EXAMPLE 1

A glass reactor equipped with a reflux condenser cooled to -70°C., an agitator and a feed line for adding reactants below the surface of the liquid reacting mass was charged with xylene. Sufficient phosgene gas from a cylinder was added below the liquid surface to give a saturated solution at the reactor temperature. During the addition of phosgene, the xylene-phosgene solution was heated to about 80°C. while maintaining a reflux of phosgene.

Then a solution of 3,4-dichloroaniline in xylene was added to the reactor by means of a 50 cc syringe with a 20 gauge needle driven by a syringe pump. The feed rate was 4.4 ml. per minute. When half the 3,4-dichloroaniline was added, a slow phosgene flow was begun to maintain an excess of phosgene and avoid formation of insoluble diaryl urea.

The temperature of the reactor was maintained at about 80°C. during the addition of the 3,4-dichloroaniline solution. Upon completing the addition of the 3,4-dichloroaniline, the temperature of the reactor was raised gradually to 135°C. Phosgene was added to maintain an excess until the reactor reached 135°C. when the flow was then stopped.

The reacting mass was maintained at 135°C. until HCl evolution ceased. The reactor was then blown with dry nitrogen above the liquid surface (i.e., not sparged) to sweep out HCl and excess phosgene. A xylene solution of the desired end product, 3,4-dichlorophenyl isocyanate, remained in the reactor.

Table 1 below summarizes data for several runs in accordance with the above procedure.

C-7001

## EXAMPLE 1 (cont'd.)

### TABLE 1

### 3,4-Dichlorophenyl Isocyanate Reactions Summary

| Run No. | DCA[1] | | Phosgene | | $T_1$°C.[2] | $T_2$°C.[3] | DCPI[4] | | |
|---|---|---|---|---|---|---|---|---|---|
| | moles | gms | moles | gms | | | gms | wt. % | yield |
| 1 | 0.5 | 81.0 | 1.05 | 104.0 | 82 | 135 | 964.8 | 9.8 | 100.5 |
| 2 | 0.3 | 48.6 | 0.70 | 69.0 | 80 | 135 | 579 | 10.2[5] | >98[5] |
| 3 | 0.5 | 81.0 | 1.28 | 127.0 | 80 | 135 | 961.7 | 10.2[5] | >98[5] |
| 4 | 0.5 | 81.0 | 1.05 | 104.0 | 79 | 135 | 965.0 | 10.2[5] | >98[5] |

[1] 3,4-dichloroaniline

[2] Temperature at which the reactor was maintained during the addition of DCA.

[3] Temperature to which the reactor was raised after the addition of DCA was completed.

[4] 3,4-dichlorophenyl isocyanate.

[5] The end product of Runs 2, 3 and 4 were combined. The values of 10.2 wt. % and >98% yield are the averages of the three runs.

C-7001

## EXAMPLE 2

A 30 wt. % xylene solution of 3,4-dichloroaniline was added to excess phosgene (100-300%) in xylene to give a 10 wt. % xylene solution of 3,4-dichlorophenyl isocyanate according to the procedure of Example 1. Small amounts of solids formed in this initial 10 wt. % solution. Xylene was then distilled to increase the concentration of the resulting isocyanate up to 30 wt. %. The yield was 90%. A quantity of solids appeared upon concentration, corresponding to 10% of diaryl urea.

## EXAMPLE 3

A 30 wt. % xylene solution of 3-chloro-4-methylaniline was added to excess phosgene (100-300%) in xylene to give a 10 wt. % xylene solution of 3-chloro-4-methylphenyl isocyanate according to the procedure of Example 1. Xylene was then distilled to increase the concentration of the resulting isocyanate up to 31.9 wt. %. No residual phosgene was detected. No solids were formed in the process.

C-7001

0143501

What is Claimed is:

1. A process for producing an organic isocyanate comprising:

a) charging a mixture of a primary monoamine and an inert liquid diluent into a reaction mixture comprising an inert organic liquid diluent and at least a stoichiometric excess of phosgene while maintaining the reaction temperature at from about 75°C. up to about 100°C. and feeding phosgene to saturate the liquid phase; and

b) heating and refluxing the phosgene saturated reaction mixture after the charging of the primary monoamine is completed up to about 150°C. while feeding phosgene so that the organic isocyanate is formed.

2. The process of Claim 1 wherein the primary monoamine is aniline.

3. The process of Claim 1 wherein the primary monoamine is haloaniline.

4. The process of Claim 3 wherein the haloaniline is 3,4-dichloroaniline.

5. The process of Claim 1 wherein the primary monoamine is an alkylaniline.

6. The process of Claim 1 wherein the primary monoamine is a haloalkylaniline.

7. The process of Claim 6 wherein the haloalkylaniline is 3-chloro-4-methylaniline.

8. The process of Claim 1 wherein the reaction temperature of step a) is maintained at from about 75°C. up to about 85°C.

C-7001